# EUROPEAN PATENT APPLICATION

(11) **EP 1 402 816 A1**
(43) Date of publication of application: **31.03.2004**
(21) Application number: 02360278.2
(22) Date of filing: 27.09.2002
(51) Int. Cl.: A61B 5/117

(54) **Method for rendering fingerprints visible**

(71) Applicant: Communauté Européenne de l'Energie Atomique (EURATOM), 1049 Bruxelles (BE)
(72) Inventor: Ray, Ian, 76646 Bruchsal (DE)
(74) Representative: Freylinger, Ernest T.

(57) **Abstract**

The present invention concerns a method for rendering fingerprint(s) visible by subjecting or exposing the surface of the substrate presumably bearing the fingerprint(s), or at least a corresponding region of said surface, to a fuming agent, under adapted conditions.

Said method is characterized in that said fuming agent consists of an agent able to react with the fatty substances of the fingerprints and incorporating an additive substance able to mark a film-like material coated or impregnated with an adapted reactive substance, and in that said method also consists in then placing a portion of such a film-like material in a close position to said surface or surface region and, possibly, submitting said exposed portion of said film-like material to a developing or visualisation process.

## Description

The present invention is related to investigation and evidence collecting techniques in connection with fingerprints, and concerns a method for rendering fingerprints visible, especially on uneven and discontinuous substrates.

In spite of modem developments in forensic science involving DNA fingerprinting and the analysis of other human traces, the conventional fingerprint remains the main tool for linking a suspect to the scene of a crime or for identifying with a high certainty the perpetrator of a crime.

Various techniques have been developed for rendering fingerprints visible, enabling them to be recorded photographically. On a flat smooth solid surface, such as a pane of glass or a metal plate, this is relatively easily performed using a fine powder which can be coloring or fluorescent.

A known alternative method is to use cyanoacrylate as a fuming agent (see for example US-4 297 383). Under the correct conditions of temperature and humidity, this agent reacts with the fats left behind in the impression of the fingerprint, to form a polymer. This latter can then be made visible by dusting with a powder or by the use of certain fluorescent dyes. This again works very efficiently with a flat solid surface.

However, difficulties are encountered in the case of uneven or fibrous surfaces such as paper and cloth, because dusting or dying of the latent fingerprint is hindered by the open porous nature of the surface.

This marking operation can be done with some difficulty on paper. But there is no effective solution at the moment for developing fingerprint images on cloth.

If this was possible it would represent a major advance in the investigation and resolution of serious crimes such as murder and assault where contact with clothing is often involved.

The aim of the present invention is to overcome the afore mentioned limitation and to propose a solution enabling good fingerprint rendering on various materials and surfaces, especially on uneven and discontinuous substrates such as paper, fabric and clothing material.

To achieve this goal the present invention concerns a method for rendering fingerprint(s) visible by subjecting or exposing the surface of the substrate presumably bearing the fingerprint(s), or at least a corresponding region of said surface, to a fuming agent, under adapted conditions.

The inventive method is mainly characterized in that said fuming agent consists of an agent able to react with the fatty substances of the fingerprints and incorporating an additive substance able to mark a film-like material coated or impregnated with an adapted reactive substance, and in that said method also consists in placing a portion of such a film-like material in a close position to said surface or surface region and, possibly, submitting said exposed portion of said film-like material to a developing or visualisation process. Said latter step is not required if the film material is self developing.

The size of the used film-like material will be of course equivalent to the area of the substrate with suspected fingerprints.

According to a prefered embodiment of the invention, the fuming agent is a cyanoacrylate based agent, said additive comprising advantageously a radioactive isotope and said film-like material comprising advantageously a photographic film, said latter being for example usually available film material.

Although many radioactive isotopes could be used as additives in the present invention, a natural harmless isotope such as radioactive ¹⁴C is prefered.

In order to obtain a high resolution in terms of reproduction of the fingerprint(s) details beared by the analysed surface of the substrate, said material is advantageously brought into close or intimate contact with the surface or surface region bearing the fingerprint(s), during a given exposure time depending on the used film-like material.

Thus, according to a prefered materialisation of the inventive method, said latter proposes to label cyanoacrylate with radioactive ¹⁴C , and to use this labeled cyanoacrylate as a fuming agent for fingerprints on difficult substrates. This eliminates the step of developing the latent fingerprint(s) with soot or dyes, since said latent fingerprint(s) can then be imaged directly by placing a photographic film in close contact with the substrate.

The method can also be applied to the imaging of fingerprints on curved or irregular surfaces.

The present invention als concerns the use of radioactively-labelled cyanoacrylate as a fuming agent in a method for rendering fingerprints visible, in connection with a film-like imaging material, especially within the context of the before described method.

Preferably, the radioactive labelling additive is ¹⁴C.

The present invention is, of course, not limited to the preferred embodiments described and represented herein, changes can be made or equivalents used without departing from the scope of the invention.

## Claims

1. Method for rendering fingerprint(s) visible by subjecting or exposing the surface of the substrate presumably bearing the fingerprint(s), or at least a corresponding region of said surface, to a fuming agent, under adapted conditions, **characterized in that** said fuming agent consists of an agent able to react with the fatty substances of the fingerprints and incorporating an additive substance able to mark a film-like material coated or impregnated with an adapted reactive substance, and **in that** said method also consists in then placing a portion of such a film-like material in a close position to said surface or surface region and, possibly, submitting said exposed portion of said film-like material to a developing or visualisation process.

2. Method according to claim 1, **characterized in that** the fuming agent is a cyanoacrylate based agent.

3. Method according to claim 1 or 2, **characterized in that** said additive comprises a radioactive isotope and said film-like material comprises a photographic film.

4. Method according to any of claims 1 to 3, **characterized in that** said additive is radioactive ¹⁴C.

5. Method according to any of claims 1 to 4, **characterized in that** said film-like material is brought into close contact with the surface or surface region bearing the fingerprint(s), during a given exposure time.

6. Use of radioactively-labelled cyanoacrylate as a fuming agent in a method for rendering fingerprints visible, in connection with a film-like imaging material.

7. Use according to claim 6, **characterized in that** the radioactive labelling additive is ¹⁴C.
